# EUROPEAN PATENT APPLICATION

(11) **EP 1 281 401 A2**
(43) Date of publication of application: **05.02.2003**
(21) Application number: 02014495.2
(22) Date of filing: 29.06.2002
(51) Int. Cl.: A61K 35/78

(54) **The medical effect of Jojoba oil**

(30) Priority: 07.07.2001 EG 75001
(71) Applicant: South Egypt Drug Industries Co (SEDICO), S.A.E. Co, 6 October City (EG); Egyptian Natural Oil Co (Natoil) S.A.E. CO, 10 Ramadan City (EG)
(72) Inventor: El Mogy, Nabil Sadek, Nasr City - Cairo (EG)
(74) Representative: Meissner, Bolte & Partner

(57) **Abstract**

Jojoba oil, a natural product, is characterized by being non-irritant and non-allergic to skin and mucous membrane. This oil has lubricant, moisturizing and soothing properties, as well as an anti-bacterial, anti-inflammatory, and anti-oxidant properties. It has a very high healing power and improves blood circulation, and also has high penetration into stratum cornium.

## Description

### Previous Art:

Jojoba oil was used in Lubricating motors and added to cosmetic preparations.

### The Problem and The Points of Technical Dificiency

There is no problem

That the previous uses of Jojoba are not related to the new uses to be protected.

### Detailed Description

Natural Jojoba oil is extracted from seeds of Jojoba shrub {Simmondsia Chinensis (LINK) Schnieder}. It is made up of straight chain esters of mono-unsaturated long chain fatty acids and fatty alcohols and has an average total carbon chain length of 42 carbons, so it is classified chemically as a liquid wax.

Jojoba oil is composed entirely of esters of high molecular weight, straight chain mono-ethylenic acids and mono-ethylenic alcohols.
The unsaturated acids are mixture of eicosanoic (C₂₀) and docosanoic (C₂₂), with small quantities of palmiloleic (C₁₈) and oleic (C₁₆) acids. The unsaturated alcohols are a mixture of eicosanol and docosanol, with smaller quantities of hexacosanol and alcohols of lower molecular weight.

Jojoba oil is non-volatile and free from rancidity, and this is attributed in part to presence of tocopherols (natural anti-oxidant) as a minor constituent and mainly to its wax structure (It has only one alcohol group, so it is not subject to oxidation and will never become rancid). Furthermore, researches showed that Jojoba oil possesses the six general factors which increase permeation into stratum comium :- low viscosity, high unsaturation, low saponification number, short carbon chain length, low lecithin (Jojoba oil has no lecithin), straight chain molecule and branched esters.

Jojoba oil helps to avoid the two important causes of premature aging of the skin :- dryness and lipid perioxidation. It serves as an excellent non-occlusive moisturizing agent that enhances the flexibility and suppleness of the skin. At the same time, its exceptional non-tacky spread and lubricity leave a rich velvety afterfeel.

Jojoba oil is characterized by having 50% of its content as insaponifiables (Triterpene alcohols and Phytosterols) while other vegetable oil contain only 2 ― 3 %, applying insaponifiables to skin increases elastin formation which in turn increases skin luster, elasticity and integration. Furthermore, they are skin protective, have anti-allergic properties, disinfectant, produce cell stimulation, skin regeneration and stop inflammation.

Jojoba oil was proved to have anti-inflammatory and anti-microbial effects. Five of the most common bacteria including staphylococcus aureus and pseudomonas aeruginosa as well as fungus candida albicans were not able of growing in Jojoba oil.

pH of Jojoba oil is balanced and reveals beneficial effects for dry and damaged skin.

### Extraction of Jojoba oil

1- Jojoba seeds contain 50-55% of its weight oil.
2- Jojoba oil is extracted in phase one by pressing the crashed seeds under pressure reaching gradually from 1 to 35-38 Ton/Inch.
3- 26-28% oil from the seeds weight is obtained form the first pressing phase, and called phase one oil, which used for medical application.
4- After separating and grinding the residual squeezed seeds are pressed again under pressure up to 100 Ton/Inch for extracting second phase oil, which is used in cosmetic and other fields applications. Also it is called second phase oil.
5- Independently, the oils are (first-phase-oil and second-phase -oil) elutriated for certain time.
6- Oils are passed through 4 level filter. Then oils are left for elutriation for certain time.
7- Oils are refiltered through 4-level filter, higher than the first 4-level filter, to obtain the final oil for medical use.
8- The extracted oil in the first phase is superior to the ordinary oil especially in the medical effect.

### The New in Subject :

Jojoba oil is used in the treatment of the following diseases :

### A- Dermal Diseases :

**A-1 It has been effectively used with salicylic acid in the treatment of psoriasis, dandruff, acne and fish skin disease.**
A-2 It has been effectively used with zinc oxide in the treatment of napkin dermatitis, skin rash, allergic dermatitis and monilial infection.
A-3 Treatment of insect bite.
A-4 Treatment of fungal infect in toes.
A.5 Protecting skin from non-infective allergic materials such as liquid washing soap.
A-6 It has been effectively used in treatment of mange.
A-7 Treating first degree burns and sun burns.
A-8 Protecting skin from hazards of ultra violate rays during suntan.
A-9 As a first aid for wounds and treating inflammatory wounds and enhancing healing by primary intention.
A-10 Treatment of bedsores.
A-11 It has been used in assisting treatment of alopecia areata and rogenetica.
A-12 Eliminating hair lice.
A-13 It has been effectively used in treatment of Herpes simplex labialis (cold sores).
A-14 It has been effectively used in healing diabetic wounds specially diabetic foot.

### B- Jojoba oil is used in treatment of gingivostomatitis and toothache :

B-1 It has been effectively used with lidocaine in the treatment of recurrent aphthous ulceration.
B-2 It has been used in toothpaste to treat gingivites and improve the circulation of gingiva.
B-3 It has been effectively used with zinc oxide in temporary filling.
B-4 It has been effectively used in the treatment of the dry socket as a side effect of chemotherapy and radiotherapy.

### C- Rheumatic pain and arthritis :

C-1 Treating and reliving pains of polyarthitis, rheumatic pains, arthralgia, and spondylitis.
C-2 Enhancing the effect of physiotherapy and supporting body to regain its physical ability in addition to eliminating the stress and cramps.

### D-It has been effective in treatment of otitis externa and otitis media :

### E- Treatment of eye diseases :

E-1 It has been effectively used in treatment of thrombosis.

### F- General surgery :

F-1 It has been effectively used as suppositories in treatment of acute and chronic anal fissure and anal fistula.
F-2 Treatment of prostatits.
F-3 Treatment of Hiemorroids (at 2^{nd} degree).
F-4 Treatment of early stages of Varicose Vein.

### G- Gynecology of Obstetrics :

G-1 Vaginal suppository in treatment of vaginitis.
G-2 Reliving the pain accompanied with dysmenorrhoea
G-3 Treatment of nipple crakes and improve its healing.

### The method of use :

Jojoba oil is used by being added to other medicines. For achieving better effects of the novel medicine without any side effects. Examples are as following :
1- 10% Of Jojoba oil, and 0.5% of Lidocaine hydrochloride are used for treating the inflammation of mucous membrane of mouth and condition resulted form administration of antibiotics for long time.
2- 50% Of Jojoba, and 2% salicylic acid are used in ointment form for treating acne, psoriases, dandruff, sebum (abnormal excessive exudatation of sebaceous glands in skin) and fish skin disease (Thick epidermis being tough and continually scalling)
3- 50% Of Jojoba oil with 8% Of zinc oxide can be used in treating conditions of skin rash and allergic dermatitis.
4- As suppository contain 20% of Jojoba oil can be used to treat the anal fissure.
5- Vaginal suppository containing 20% of Jojoba oil can be used in treating non-infective vaginitis.

## Claims

1. Jojoba oil is extracted by pressing the crashed seeds gradually under pressure form 1 to 35 ― 38 Ton/Inch to obtain first phase oil, then meal well be crashed again and pressed under pressure up to 100 Ton/Inch to obtain second phase oil, then each type of oil will go through different levels of filtration, since the first phase oil is used for medical application for treating many illnesses.

2. As mentioned in the first claim, from seeds, Jojoba oil and its derivatives are extracted including straight chains of mono-ethylenic acids and mono ethylenic alcohols . further more Jojoba oil is non-volatile, not subject to oxidation and not liable to rancidity in addition to its high penetrability to skin as a result of its low viscosity, high unsaturation, low saponification number, short carbon chain length, low lecithin, straight chain molecule and branched esters.

3. As motioned in the first claim, the jojoba oil is used as anti-bacteria, skin protective, anti-allergic, anti-inflammatory, anti-fungal, improving wound healing and blood circulation in the skin.

4. As mentioned in the first claim, Jojoba oil as an essential element, is used for treating many dermal diseases.

5. As mentioned in the first claim, Jojoba oil is used as an essential element, in the treatment of the inflammation of mucus membrane of the mouth, temporary filling and dry sockets.

6. As mentioned in the first claim, Jojoba oil is used as a main element in treating and reliving pains of polyarthitis, rheumatic pains, arthralgia, and spondylitis.

7. As mentioned in the first claim, Jojoba oil is used as a main element, for treating otitis externa and otitis media.

8. As mentioned in the first claim, as a main element, Jojoba oil is used in treatment of thrombosis.

9. As mentioned in the first claim, as a main element, Jojoba oil is used in treatment of some general surgeries such as anal fissure, anal fistula, prostatits, and Hiemorroids (at 2^{nd} degree).

10. As mentioned in first claim, Jojoba oil is used as a main element in treatment of vaginitis, and nipple crakes in addition to reliving the pain accompanied with dysmenorrhoea.

11. As in the first claim, compound contain 10% of Jojoba oil and 0,5% Of lidocaine hydrochloride, is particularly effective in treating mouth ulcer and gingivostomatitis and removing the erythena in addition to reducing Odema associated to these inflammations ― this effect returns to the Jojoba oil properties as anti-bacterial and anti-inflammation substance. Also, this oil acts as a painkiller for that conditions, Some of that oil's indications are : gingivostomatitis and conditions resulted from using antibiotics for long time moreover, this compound has no side effects.

12. As mentioned in the first claim, preparation contain 50% of Jojoba oil and 2%. Of salicylic acid is used for treating acne, psoriasis dandruff sebum (abnormal) excessive exudatation of seba ceous glands in skin and fish skin disease it could be used in treatment of fungal infection as tinia. This preparation also has no side effect.

13. As in the first claim, suppository contain 20% jojoba oil can be used to treat the anal fissure. It could relieve the intensity of pains and the complications in many patients, also it relieves pills patients. Moreover, this suppository has no side effects.

14. As in the first claim, ointment contains 50% of Jojoba oil with 8% of zinc oxide used in treating condition of rash, napkin dermatitis, skin inflammation, insect bytes, monilyases, and nipple fissure, no side effects have been resulted from this preparation.

15. As in the first claim, a vaginal suppository containing 20% Jojoba oil is used as highly effective material to eliminate the vaginitis (acute or recurrent). This suppository has no side effects.
Form all the above mentioned benefits, it becomes clear that Jojoba oil, in its all forms and cases, all the illnesses treated with it, has not any side-effects on the human body or environment.
